# EUROPEAN PATENT SPECIFICATION

(11) **EP 3 320 781 B1**
(45) Date of publication and mention of the grant of the patent: **10.06.2020**
(21) Application number: 16821395.7
(22) Date of filing: 05.07.2016
(51) Int. Cl.: A23C 9/12, A23C 9/13, A23C 19/055, C12N 9/58, C12N 15/09, A23C 19/032, C12N 9/64, A23C 9/127, A23C 9/123

(54) **DAIRY PRODUCT**
MILCHPRODUKT
PRODUIT LAITIER

(30) Priority: 06.07.2015 JP 2015135591
(43) Date of publication of application: 16.05.2018
(73) Proprietor: Godo Shusei Co., Ltd., Tokyo 104-8162 (JP)
(72) Inventor: OGASAWARA, Junki, Matsudo-shi Chiba 271-0064 (JP); HORIGUCHI, Hirofumi, Matsudo-shi Chiba 271-0064 (JP)
(74) Representative: Schiener, Jens
(86) International application number: PCT/JP2016/069859
(87) International publication number: WO 2017/006926

(56) References cited:
- WO-A2-2011/075677
- ES-A1- 2 303 760
- JP-A- 2004 033 093
- JP-B2- H0 218 834
- US-A1- 2005 095 316
- US-A1- 2005 095 317
- SATYENDRA K. GARG. ET AL.: 'Rennet: Current Trends and Future Research' FOOD REVIEWS INTERNATIONAL vol. 10, 1994, pages 313 - 355, XP009508177
- DATABASE PROTEIN [Online] 15 March 2014 XP055511662 Retrieved from NCBI Database accession no. XP_006964971

### SEQUENCE LISTING

<110> GODO SHUSEI CO., LTD.
<120> Dairy Products
<130> GD0012
<150> JP 2015-135591
   <151> 2015-07-06
<160> 5
<170> PatentIn version 3.5
<210> 1
   <211> 395
   <212> PRT
   <213> Trichoderma reesei
<400> 1
   Met Lys Ser Ala Leu Leu Ala Ala Ala Ala Leu Val Gly Ser Ala Gln
   1               5                   10                  15
   Ala Gly Ile His Lys Met Lys Leu Gln Lys Val Ser Leu Glu Gln Gln
               20                  25                  30
   Leu Glu Gly Ser Ser Ile Glu Ala His Val Gln Gln Leu Gly Gln Lys
           35                  40                  45
   Tyr Met Gly Val Arg Pro Thr Ser Arg Ala Glu Val Met Phe Asn Asp
       50                  55                  60
   Lys Pro Pro Lys Val Gln Gly Gly His Pro Val Pro Val Thr Asn Phe
   65                  70                  75                  80
   Met Asn Ala Gln Tyr Phe Ser Glu Ile Thr Ile Gly Thr Pro Pro Gln
                   85                  90                  95
   Ser Phe Lys Val Val Leu Asp Thr Gly Ser Ser Asn Leu Trp Val Pro
               100                 105                 110
   Ser Gln Ser Cys Asn Ser Ile Ala Cys Phe Leu His Ser Thr Tyr Asp
           115                 120                 125
   Ser Ser Ser Ser Ser Thr Tyr Lys Pro Asn Gly Ser Asp Phe Glu Ile
       130                 135                 140
   His Tyr Gly Ser Gly Ser Leu Thr Gly Phe Ile Ser Asn Asp Val Val
   145                 150                 155                 160
   Thr Ile Gly Asp Leu Lys Ile Lys Gly Gln Asp Phe Ala Glu Ala Thr
                   165                 170                 175
   Ser Glu Pro Gly Leu Ala Phe Ala Phe Gly Arg Phe Asp Gly Ile Leu
               180                 185                 190
   Gly Leu Gly Tyr Asp Thr Ile Ser Val Asn Gly Ile Val Pro Pro Phe
           195                 200                 205
   Tyr Gln Met Val Asn Gln Lys Leu Ile Asp Glu Pro Val Phe Ala Phe
       210                 215                 220
   Tyr Leu Gly Ser Ser Asp Glu Gly Ser Glu Ala Val Phe Gly Gly Val
   225                 230                 235                 240
   Asp Asp Ala His Tyr Glu Gly Lys Ile Glu Tyr Ile Pro Leu Arg Arg
                   245                 250                 255
   Lys Ala Tyr Trp Glu Val Asp Leu Asp Ser Ile Ala Phe Gly Asp Glu
               260                 265                 270
   Val Ala Glu Leu Glu Asn Thr Gly Ala Ile Leu Asp Thr Gly Thr Ser
           275                 280                 285
   Leu Asn Val Leu Pro Ser Gly Leu Ala Glu Leu Leu Asn Ala Glu Ile
       290                 295                 300
   Gly Ala Lys Lys Gly Phe Gly Gly Gln Tyr Thr Val Asp Cys Ser Lys
   305                 310                 315                 320
   Arg Asp Ser Leu Pro Asp Ile Thr Phe Ser Leu Ala Gly Ser Lys Tyr
                   325                 330                 335
   Ser Leu Pro Ala Ser Asp Tyr Ile Ile Glu Met Ser Gly Asn Cys Ile
               340                 345                 350
   Ser Ser Phe Gln Gly Met Asp Phe Pro Glu Pro Val Gly Pro Leu Val
           355                 360                 365
   Ile Leu Gly Asp Ala Phe Leu Arg Arg Tyr Tyr Ser Val Tyr Asp Leu
       370                 375                 380
   Gly Arg Asp Ala Val Gly Leu Ala Lys Ala Lys
   385                 390                 395
<210> 2
   <211> 395
   <212> PRT
   <213> Trichoderma virens Gv29-8
<400> 2
   Met Lys Ser Ala Leu Ile Ala Ala Ala Ala Leu Val Gly Ser Ala Gln
   1               5                   10                  15
   Ala Gly Val His Lys Met Lys Leu Gln Lys Val Ser Leu Glu Gln Gln
               20                  25                  30
   Leu Glu Gly Ser Thr Ile Glu Ser Gln Val Gln His Leu Gly Gln Lys
           35                  40                  45
   Tyr Met Gly Val Arg Pro Thr Ser Arg Ala Asp Val Met Phe Asn Asp
       50                  55                  60
   Lys Leu Pro Lys Ile Gln Gly Gly His Pro Val Pro Val Thr Asn Phe
   65                  70                  75                  80
   Met Asn Ala Gln Tyr Phe Ser Glu Ile Thr Ile Gly Thr Pro Pro Gln
                   85                  90                  95
   Thr Phe Lys Val Val Leu Asp Thr Gly Ser Ser Asn Leu Trp Val Pro
               100                 105                 110
   Ser Gln Ser Cys Asn Ser Ile Ala Cys Phe Leu His Ala Thr Tyr Asp
           115                 120                 125
   Ser Ser Ser Ser Ser Thr Tyr Lys Gln Asn Gly Ser Asp Phe Glu Ile
       130                 135                 140
   His Tyr Gly Ser Gly Ser Leu Thr Gly Phe Ile Ser Asn Asp Val Val
   145                 150                 155                 160
   Thr Ile Gly Asp Leu Lys Ile Gln Lys Gln Asp Phe Ala Glu Ala Thr
                   165                 170                 175
   Ser Glu Pro Gly Leu Ala Phe Ala Phe Gly Arg Phe Asp Gly Ile Leu
               180                 185                 190
   Gly Leu Gly Tyr Asp Thr Ile Ser Val Asn Gly Ile Ile Pro Pro Phe
           195                 200                 205
   Tyr Gln Met Val Asn Gln Lys Leu Leu Asp Glu Pro Val Phe Ala Phe
       210                 215                 220
   Tyr Leu Gly Ser Gly Asp Glu Gly Ser Glu Ala Val Phe Gly Gly Val
   225                 230                 235                 240
   Asp Glu Ser His Tyr Ser Gly Lys Ile Glu Tyr Ile Pro Leu Arg Arg
   245 250 255
   Lys Ala Tyr Trp Glu Val Asp Leu Asp Ser Ile Ala Phe Gly Asp Glu
   260 265 270
   Val Ala Glu Leu Glu Asn Thr Gly Ala Ile Leu Asp Thr Gly Thr Ser
   275 280 285
   Leu Asn Val Leu Pro Ser Gly Leu Ala Glu Leu Leu Asn Ala Glu Ile
   290 295 300
   Gly Ala Lys Lys Gly Phe Gly Gly Gln Tyr Thr Val Asp Cys Ser Lys
   305 310 315 320
   Arg Asp Ser Leu Pro Asp Ile Thr Phe Ser Leu Ala Gly Ser Lys Tyr
   325 330 335
   Ser Leu Pro Ala Thr Asp Tyr Ile Ile Glu Met Ser Gly Asn Cys Ile
   340 345 350
   Ser Ser Phe Gln Gly Met Asp Phe Pro Glu Pro Val Gly Pro Leu Val
   355 360 365
   Ile Leu Gly Asp Ala Phe Leu Arg Arg Tyr Tyr Ser Val Tyr Asp Leu
   370 375 380
   Gly Lys Asn Ala Val Gly Leu Ala Lys Ala Lys
   385 390 395
<210> 3
   <211> 395
   <212> PRT
   <213> Trichoderma atroviride
<400> 3
   Met Lys Ser Ala Leu Ile Ala Ala Ala Ala Leu Val Gly Ser Ala Gln
   1 5 10 15
   Ala Gly Val His Lys Met Lys Leu Gln Lys Val Ser Leu Glu Gln Gln
   20 25 30
   Leu Glu Gly Ser Ser Ile Glu Ala Gln Val Gln Gln Leu Gly Gln Lys
   35 40 45
   Tyr Met Gly Val Arg Pro Thr Ser Arg Val Asp Val Met Phe Asn Asp
   50 55 60
   Asn Val Pro Lys Val Lys Gly Gly His Pro Val Pro Val Thr Asn Phe
   65                  70                  75                  80
   Met Asn Ala Gln Tyr Phe Ser Glu Ile Thr Ile Gly Ser Pro Pro Gln
                   85                  90                  95
   Thr Phe Lys Val Val Leu Asp Thr Gly Ser Ser Asn Leu Trp Val Pro
               100                 105                 110
   Ser Gln Ser Cys Asn Ser Ile Ala Cys Phe Leu His Ser Thr Tyr Asp
           115                 120                 125
   Ser Ser Ser Ser Ser Ser Tyr Lys Lys Asn Gly Ser Asp Phe Glu Ile
       130                 135                 140
   His Tyr Gly Ser Gly Ser Leu Thr Gly Phe Ile Ser Asn Asp Val Val
   145                 150                 155                 160
   Thr Ile Gly Asp Leu Gln Ile Lys Gly Gln Asp Phe Ala Glu Ala Thr
                   165                 170                 175
   Ser Glu Pro Gly Leu Ala Phe Ala Phe Gly Arg Phe Asp Gly Ile Leu
               180                 185                 190
   Gly Leu Gly Tyr Asp Thr Ile Ser Val Asn Gly Ile Val Pro Pro Phe
           195                 200                 205
   Tyr Gln Met Val Asn Gln Lys Leu Leu Asp Glu Pro Val Phe Ala Phe
       210                 215                 220
   Tyr Leu Gly Ser Gly Asp Glu Gly Ser Val Ala Thr Phe Gly Gly Val
   225                 230                 235                 240
   Asp Glu Ser His Tyr Ser Gly Lys Ile Glu Tyr Ile Pro Leu Arg Arg
                   245                 250                 255
   Lys Ala Tyr Trp Glu Val Asp Leu Asp Ser Ile Ala Phe Gly Asp Glu
               260                 265                 270
   Val Ala Glu Leu Glu Asn Thr Gly Ala Ile Leu Asp Thr Gly Thr Ser
           275                 280                 285
   Leu Asn Val Leu Pro Ser Gly Ile Ala Glu Leu Leu Asn Ala Glu Ile
       290                 295                 300
   Gly Ala Lys Lys Gly Tyr Gly Gly Gln Tyr Thr Ile Asp Cys Ala Lys
   305                 310                 315                 320
   Arg Asp Ser Leu Pro Asp Ile Thr Phe Ser Leu Ala Gly Ser Lys Tyr
                   325                 330                 335
   Ser Leu Pro Ala Ser Asp Tyr Ile Leu Glu Val Ser Gly Ser Cys Ile
               340                 345                 350
   Ser Thr Phe Gln Gly Met Asp Phe Pro Glu Pro Val Gly Pro Leu Val
           355                 360                 365
   Ile Leu Gly Asp Ala Phe Leu Arg Arg Tyr Tyr Ser Val Tyr Asp Leu
       370                 375                 380
   Gly Lys Gly Ala Val Gly Leu Ala Lys Ala Lys
   385                 390                 395
<210> 4
   <211> 28
   <212> DNA
   <213> Artificial Sequence
<220>
   <223> Designed primer based on Trichoderma reesei
<400> 4
   tacaaagagg ctggttccta gcttcgtc 28
<210> 5
   <211> 27
   <212> DNA
   <213> Artificial Sequence
<220>
   <223> Designed primer based on Trichoderma reesei
<400> 5
   taggactgtc ttccgctctc aaactgc 27

## Description

### Technical Field

The present invention relates to a dairy product with new texture, and a method for producing the dairy product.

### Background Art

Dairy products include one in a liquid state and one in a solid state. As the dairy product in a solid state, cheese, fermented milk (for example, yogurt), and the like have been known. Yogurt becomes solid as a result of lactic acid fermentation. Meanwhile, cheese becomes solid by action of rennet (enzyme made in mammalian stomach) that is a milk-coagulating enzyme, on casein (Patent Literature 1). In addition, as the rennet, microbially derived rennet, and genetically modified rennet are also known (Patent Literatures 1 and 2).

### Citation List

### Patent Literature

Patent Literature 1: JP 2004-33093 A
Patent Literature 2: JP hei 02-18834 B

WO2011/075677 discloses in sequence 108 a pepsin-like aspartic protease sequence from *Trichoderma reesei.* Said enzyme was not applied in dairy products.

### Summary of Invention

### Technical Problem

However, there is a problem that a dairy product coagulated with rennet generates a bitter taste. In addition, yogurt by lactic acid fermentation has insufficient solidity in many cases, and a thickening agent and a gelling agent may be mixed in commercially available yogurt by taking into consideration the shape retention during distribution and storage.

Accordingly, development of a novel dairy product that has a certain solidity and a favorable flavor is desired.

### Solution to Problem

Herein, as a result of extensive studies to find a novel milk-coagulating component to replace rennet, the present inventors have found that a protein and an analog thereof, which are predicted to be produced by a microorganism belonging to the genus Trichoderma, and which is nothing more than its amino acid sequences are predicted, have an excellent milk-coagulating action, and in particular, fermented milk obtained by the fermentation together with lactic acid bacteria or yeast has a cheese-like solidity, and becomes a dairy product having no bitterness but having a favorable flavor, and thus have completed the present invention.

That is, the present invention provides the following [1] to [7].

[1] A dairy product, including: a milk-derived rawmaterial containing casein; and a protein consisting of an amino acid sequence having 90% or more identity to the amino acid sequence represented by SEQ ID NO: 1.
[2] The dairy product according to [1], wherein the protein is a protein derived from a microorganism belonging to the genus Trichoderma.
[3] The dairy product according to [1] or [2], further including lactic acid bacteria or yeast.
[4] The dairy product according to any one of [1] to [3], wherein the dairy product is fermented milk or cheese.
[5] The dairy product according to any one of [1] to [4], wherein the dairy product has a breaking stress in a two-bite method of 5,500 Pa or more, and a breaking adhesion of 800 J/m³ or more.
[6] The dairy product according to any one of [1] to [5], not comprising a thickening agent, a stabilizer, or a gelling agent.
[7] A method for producing a dairy product, including: adding a protein consisting of an amino acid sequence having 90% or more identity to the amino acid sequence represented by SEQ ID NO: 1, and lactic acid bacteria to a milk-derived raw material containing casein; and fermenting a mixture of the protein and the lactic acid bacteria with the milk-derived raw material.

### Advantageous Effects of Invention

The dairy product of the present invention has a cheese-like solidity and imparts a favorable flavor, and is useful as a dairy product with new texture. By variously adjusting the reaction conditions between the specific protein used in the present invention and the milk-derived raw material, the dairy product of the present invention can be produced so as to have a solidity in a range of being harder than that of the conventional yogurt and softer than that of the conventional cheese.

### Brief Description of Drawings

Fig. 1 shows the temperature dependency of GODO-TCF derived protease (SEQ ID NO: 1).
Fig. 2 shows the temperature stability of GODO-TCF derived protease (SEQ ID NO: 1).
Fig. 3 shows the pH stability of GODO-TCF derived protease (SEQ ID NO: 1).
Fig. 4 shows the pH dependency of GODO-TCF derived protease (SEQ ID NO: 1).
Fig. 5 shows the effect of each enzyme at pH 5.5 to casein.
Fig. 6 shows the effect by GODO-TCF derived protease (SEQ ID NO: 1) at pH 7.2.
Fig. 7 shows the coagulability of pH-adjusted milk by GODO-TCF. Top: heat treatment, Medium: GODO-TCF 1% addition, and Bottom: GODO-TCF 10% addition.
Fig. 8 shows the changes with time of pH in yogurt during the fermentation of lactic acid bacteria are shown. (Each of arrows shows a point where the surface does not move when the tube is tilted.)
Fig. 9 shows the results of breaking strength analysis.
Fig. 10 shows the results of texture analysis under non-breaking conditions.
Fig. 11 shows the results of texture analysis under breaking conditions.

### Description of Embodiments

As the protein consisting of an amino acid sequence having 90% or more identity to the amino acid sequence represented by SEQ ID NO: 1, which is used in the dairy product of the present invention, a protein derived from a microorganism belonging to the genus Trichoderma is preferred. More specifically, for example, a functionally unknown predicted protein derived from Trichoderma reesei QM6a (available fungus described in National Center for Biotechnology Information (NCBI)) (100% identical to the amino acid sequence represented by SEQ ID NO: 1), a protein contained in a commercially available cellulase GODO-TCF derived from Trichoderma reesei (100% identical to the amino acid sequence represented by SEQ ID NO: 1), a virtual protein TRIVIDRAFT_215801 derived from Trichoderma virens Gv29-8 (available fungus described in NCBI) (94% identical to the amino acid sequence represented by SEQ ID NO: 1: SEQ IDNO: 2), a vacuolar protein A derived from Trichoderma atroviride (92% identical to the amino acid sequence represented by SEQ ID NO: 1: SEQ ID NO: 3), and a vacuolar protein derived from Trichoderma aureoviride (93% identical to the amino acid sequence represented by SEQ ID NO: 1) can be mentioned.

A more preferred protein is a protein having 92% or more identity to the amino acid sequence represented by SEQ ID NO: 1, an even more preferred protein is a protein having 93% or more identity to the amino acid sequence represented by SEQ ID NO: 1, a still more preferred protein is a protein having 94% or more identity to the amino acid sequence represented by SEQ ID NO: 1, and a yet more preferred protein is a protein having 95% or more identity to the amino acid sequence represented by SEQ ID NO: 1.

The protein used in the present invention may be extracted from a microorganism belonging to the genus Trichoderma, or may be produced by a gene recombination technique. Note that in the protein, as long as a milk-coagulating action is contained, a protein to which a sugar is bound is also included.

The protein used in the present invention has caseinolytic activity, therefore, has protease activity. Among caseins, the degradation activity to κ-casein is high the degradation activity to α-casein and β-casein is weaker than the degradation activity to κ-casein.

The protein degrades the κ-casein contained in a milk-derived raw material with a slight addition amount. According to this, α-casein (including degradation products) and β-casein (including degradation products) aggregate, and the solidity of a dairy product increases.

As the protein, a purified protein according to the claims may be used, and, for example, a culture (including a culture solution) of the microorganism belonging to the genus Trichoderma containing the protein, and an extract of the culture can be used. For example, the above-described cellulase GODO-TCF may be used as it is.

As the milk-derived raw material containing casein, which is used in the dairy product of the present invention, as long as casein is contained, for example, a liquid material or a powder material of cow milk, goat milk, sheep milk, horse milk, breast milk, or the like can be mentioned. In a case where the powdered milk is used, one mixed with, for example, water may be used. Note that in milk, raw milk, low-fat milk, fat-free milk, and processed milk are also included.

The content of the protein contained in the dairy product of the present invention may be a content with which the milk-coagulating activity is exerted. Whether or not the milk-coagulating activity is exerted varies depending on, for example, the kind of the milk-derived raw material to be used, the amount of the casein contained in the milk-derived raw material, and the production conditions (for example, reaction time, reaction temperature and reaction pH). For this reason, although it cannot be said sweepingly, as to the content of the protein, for example, if the milk-coagulating activity (caseinolytic activity) of the protein when the protein is added to a milk-derived raw material is in the range of 20 U to 300 U per 100 g of the milk raw material, a dairy product having an unprecedented solidity in the past is easily obtained.

The presence or absence of the casein degradation can be confirmed by analyzing the obtained dairyproduct by, for example, sodium dodecyl sulfate-polyacrylamide gel electrophoresis (SDS-PAGE) .

The acid protease activity of the protein contained in the dairy product of the present invention is preferably 3,000 U or less per 100 g of the milk raw material.

When the activity exceeds the level, flavor of the dairy product may be deteriorated.

It is preferred that the dairy product of the present invention further contain lactic acid bacteria or yeast from the viewpoint of the flavor and the texture . In the dairy product used the protein in combination with lactic acid bacteria, the solidity is increased as compared with that of the conventional fermented milk, therefore, it is more preferred that the dairy product of the present invention contain lactic acid bacteria.

Herein, as the Lactic acid bacteria, those belonging to the genus lactic acid bacteria, the genus Bifidobacterium, the genus Enterococcus, the genus Lactococcus, or the genus Leuconostoc can be used.

The content of lactic acid bacteria in the dairy product of the present invention is not particularly limited, however, ispreferably1, 000, 000/1mLormore, morepreferably5, 000, 000/1 mL or more, and even more preferably 10,000,000/1 mL or more.

In the dairy product of the present invention, if necessary, in addition to a sweetener such as syrup, various other food materials, for example, optional components such as various sugars, an emulsifier, and various vitamin preparations can be mixed. Specifically, a sugar such as sucrose, glucose, fructose, palatinose, trehalose, lactose, xylose, and maltose; a sugar alcohol such as sorbitol, xylitol, erythritol, lactitol, palatinit, reduced sugar syrup, and reduced maltose syrup; a high-intensity sweetener such as aspartame, thaumatin, sucralose, acesulfame K, and stevia; an emulsifier such as a sucrose fatty acid ester, a glycerine fatty acid ester, a polyglycerol fatty acid ester, a sorbitan fatty acid ester, and lecithin; milk fat such as cream, butter, and sour cream, an acidulant such as citric acid, lactic acid, acetic acid, malic acid, tartaric acid, and gluconic acid; various vitamins such as vitamin A, vitamins B, vitamin C, and vitamins E; minerals such as calcium, magnesium, zinc, iron, and manganese; and various flavors can be mixed.

The dairy product of the present invention has sufficient solidity due to the milk-coagulating action of the protein, therefore, it is not required to contain a coagulation component such as a thickening agent, a stabilizer, a gelling agent, and an adhesive paste, and it is preferred not to substantially contain such a coagulation component.

The dairy product of the present invention can be produced in accordance with a conventional method for producing fermented food by mixing the milk-derived raw material and the protein, and milk-coagulating the resultant preparation, or by mixing the milk-derived raw material, the protein, and lactic acid bacteria or yeast. As a method for producing fermented food, a method in which the milk-derived raw material and the protein are mixed, lactic acid bacteria or yeast is inoculated into the resultant preparation and the inoculated preparation is cultured, and after homogenization of the cultured preparation, for example, an optional component such as a syrup liquid, and a flavor are added can be mentioned.

The dairy product obtained according to the present invention has a specific texture of having a solidity that is higher than the solidity of the yogurt in a solid state, and further has favorable flavor without having, for example, a bitter taste, in spite of containing no thickening agent or the like. Such a texture is a specific effect due to the protein described above.

In the dairy product of the present invention, preferably, the breaking stress is 5, 500 Pa or more and the breaking adhesion is 800 J/m³ or more in a two-bite method, in view of the texture, and further the breaking stress is more preferably 5, 500 to 12 , 000 Pa, and the breaking adhesion is more preferably 800 to 1,200 J/m³.

As the preferred form of the dairy product of the present invention, fermented milk, cheese, ice cream, ice milk, and lacto-ice can be mentioned, but fermented milk and cheese are more preferred. As the fermented milk, a form of solid-type yogurt is particularly preferred.

The protein acts on casein and shows a milk-coagulating action, therefore is useful as a milk-coagulating agent for a dairy product.

### Examples

Next, the present invention will be described by way of examples.

### Production Example 1

### (1) (Separation of protease from GODO-TCF)

100 mL of GODO-TCF (mutant of Trichoderma reesei QM6a, manufactured by GODO SHUSEI CO., LTD.) was concentrated to about 50 mL with a UF pencil-type module API-0013 (molecular weight cutoff of 6,000), and into the concentrated preparation, 150 mL of a 20 mM MES buffer solution (pH 6.0) was added. This procedure was repeated four times.

The obtained concentrate was applied to a Giga Cap Q-650M (TOYOPEARL) column equilibrated with a 20 mM MES buffer solution (pH6.0), and a fraction containing the milk-coagulating activity eluted with a linear gradient of 0-0.5 M NaCl was recovered. Into the recovered active fraction, ammonium sulfate (hereinafter, referred to as AS) was added so that the final concentration is 0.5 M, and then the resultant preparation was applied to a Butyl-650 M (TOYOPEARL) column equilibrated with a 20 mM MES buffer solution (pH 6.0) containing 0.5 M AS, and eluted with a linear gradient of 0.5-0 M AS. Then, the active fraction was recovered, and concentrated with Amicon Ultra™-30K (molecular weight cutoff of 30 kDa) (4,000 × g, 120 minutes), and then the resultant preparation was applied to a Hiprep 16/60 Sephacryl S-200 HR column (GE Healthcare) equilibrated with a 20 mM MES buffer solution (pH 6.0) containing 0.15 M NaCl, and the active fraction was recovered.

When the active fraction was subjected to SDS-PAGE, a single band was confirmed at around 33 kDa.

### (2) Amino acid sequencing of GODO-TCF derived protease

The activated fraction eluted by Butyl-650 M column chromatography in the purification process described in (1) was subjected to SDS-PAGE, and a target enzyme band at about 33 kDa was excised, and then from a sequence of a peptide fragment, gene sequence information of a predicted protein [Trichoderma reesei QM6a] having high homology of the amino acid sequence from the NCBInr database using a Mascot seach program was obtained. On the basis of the obtained gene information, PCR primers 5' -tacaaagaggctggttcctagcttcgtc-3' (Foward: SEQ ID NO: 4), and 5'-taggactgtcttccgctctcaaactgc-3' (Reverse: SEQ ID NO: 5) were designed, and using a genomic DNA of a Trichoderma reesei QM9414 strain as a template, the target gene was amplified with EX Taq (TaKaRa). The amplified DNA fragment of about 1,600 bp was excised, and purified using FastGene Gel/PCR Extraction (NIPPON Genetics Co, Ltd), and then the DNA sequence information was obtained, and the amino acid sequence was determined by a translation program. The obtained amino acid sequence was shown as SEQ ID NO: 1. The amino acid sequence was 100% identical to the amino acid sequence of Trihoderma reesei QM6a.

### (3) Other properties of GODO-TCF derived protease

### (Method)

An enzyme sample of the eluted active fraction of Butyl-650M during the purification process described in (1) was used. The temperature dependency was determined using chymosin activity at 20, 30, 40, 45, 50, 55, 60, or 65°C as an indicator. Measurement of chymosin activity was performed by a method in which the International Committee for Standardization in Hematology was partially modified. The substrate solution was prepared by dissolving low-heat, low- fat spray dried milk powder at a concentration of 11 g/100 mL, adding CaCl₂ so as to have a final concentration of 3 mM, and then adjusting the pH to 5.5 with 1 N hydrochloric acid. Into 0.1 mL of the enzyme solution preincubated at a measurement temperature, 5 mL of the substrate solution preincubated in the same manner was added, and the resultant preparation was stirred so as not to foam. After that, a test tube was tilted around every one minute to check whether coagulated milk adheres to the wall surface, while conducting the reaction at each temperature, and the time when the coagulated milk adheres for the first time was measured. The measurement was performed at the same time as the measurement of a reference standard with a known measurement value, and each milk-coagulating time was examined and the activity level was calculated.

As to the temperature stability, the enzyme solution was incubated at 4, 20, 30, 40, 50, 60, or 70 for 2 hours, and then the remaining chymosin activity after diluting the incubated preparation by 2 times with a 100 mM MES buffer (pH 5.5, r.t.) was measured in accordance with the activity measurement method described above.

As to the pH stability, the enzyme solution was diluted by 3 times with each buffer at pH 3.0, 3.5, 4.0, 4.5, 5.0, 5.5, 6.0, 6.5, 7.2, 7.5, 8.0, 8.5, or 9.0 (pH 3.0 to 6.0: 0.2 M citric acid-Na citrate buffer, pH 6.0 to 8.0: 0.2M phosphoric acid-Na phosphate buffer, and pH 7.2 to 9.0: Tris-HCl buffer), and the diluted preparation was left to stand at 4°C for 6 hours. Then, the resultant preparation was diluted by 2 times with a 100 mM MES buffer (pH 5.5, r.t.), and the remaining chymosin activity was measured in accordance with the activity measurement method described above.

The pH dependency was evaluated by a method using a releasing activity of acid-soluble peptides. As to the substrate solution, 1.2 g of milk casein (CALBIOCHEM) was weighed, and to the milk casein, 20 mL of distilled water and 20 mL of a 0.1 M Tris solution were added and dissolved, and then the resultant preparation was heated at 60°C for 10 minutes in a boiling bath. After that, into the heated preparation, 100 mL of each 500 mM buffer (pH 3.0 to 6.0: 0. 2 M citric acid-Na citrate buffer, pH 6. 0 to 8.0: 0.2 M phosphoric acid-Na phosphate buffer, and pH 7.2 to 9.0: Tris-HCl buffer), was added, and each pH was adjusted with 1 N hydrochloric acid or sodium hydroxide, and then the resultant preparation was filled up to 200 mL with distilled water to perform the preparation. In the reaction, 0.5 mL of the enzyme solution was preincubated at 50°C for 3 minutes, 2.5 mL of the substrate solution was added to the preincubated preparation, and the resultant preparation was thoroughly stirred and reacted at 50°C for 10 minutes. After that, 1. 8 g of trichloroacetic acid, 11.45 g of acetic anhydride, and 21 mL of acetic acid were mixed, into the mixture, 2.5 mL of a precipitation reagent that had been adjusted to 1 L with distilled water was added, and the resultant preparation was left to stand at room temperature for about 5 minutes. After that, a filtrate obtained by filtering the resultant preparation through a ADVANTEC No.4A filter paper was measured for the absorbance in 275 nm, and the activity level was calculated using the enzyme level at which acid-soluble low molecular weight degradation products corresponding to 1 µg of tyrosine per minute are generated as 1 unit.

### (Results)

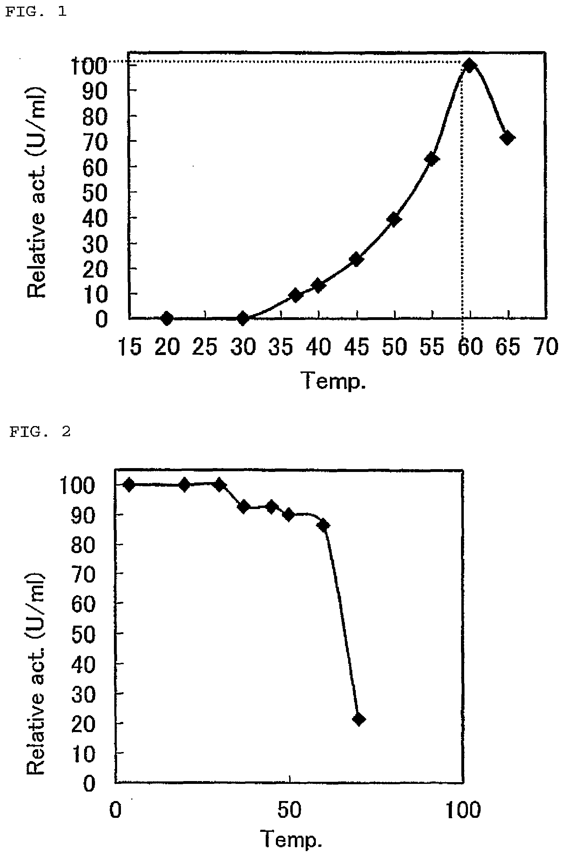

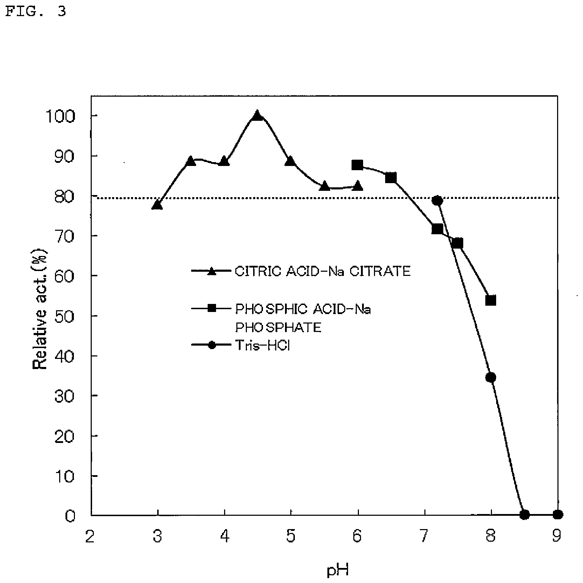

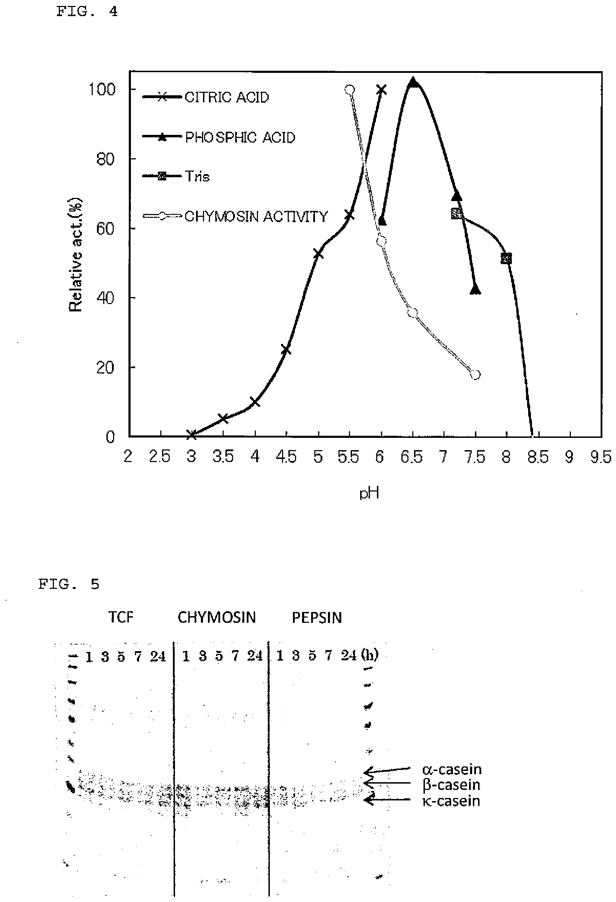

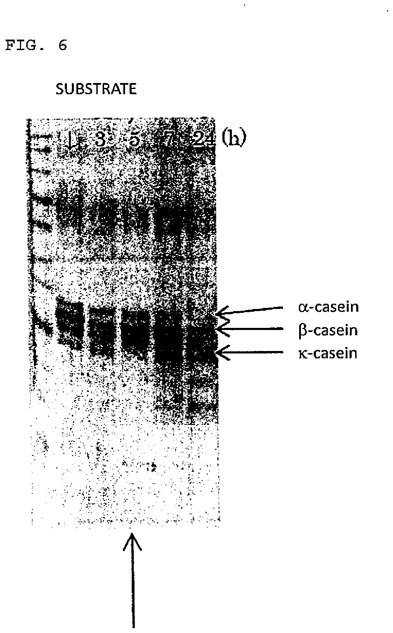

(1) The temperature dependency of the GODO-TCF derived protease (the protease represented by SEQ ID NO: 1) is shown in Fig. 1, the temperature stability of the GODO-TCF derived protease is shown in Fig. 2, the pH stability of the GODO-TCF derived protease is shown in Fig. 3, and the pH dependency of the GODO-TCF derived protease is shown in Fig. 4. As shown in Figs. 1 to 4, the GODO-TCF derived protease showed the maximum activity at about 60°C, and maintained the residual activity of 80% or more at pH 3.5 to 6.5 and up to 60°C. In the pH dependency, it can be assumed that the optimum pH of chymosin activity is on the acidic side from 5.5, whereas it can be understood that the acid-soluble peptide releasing activity is in the vicinity of pH 6.5.
(2) The effect of GODO-TCF derived protease to casein at pH 5.5, and the effect of the chymosin and pepsin contained in rennet on casein were examined. The effect of GODO-TCF-derived protease to casein at pH 7.2 was examined. The results are shown in Figs. 5 and 6.

As a result, it was suggested that the GODO-TCF derived protease has high specificity for κ-casein at pH 5.5 (Fig. 5), whereas further acts to α and β-caseins in the vicinity of pH 7.2 (Fig. 6). It was confirmed that chymosin has high specificity for κ-casein, and pepsin has a thinner band as a whole. It was found that when comparing the GODO-TCF derived protease with the other two proteases, the specificity of the GODO-TCF milk coagulating protease was κ-casein > α-casein.

### Example 1

The pH was adjusted to 5.9 with 10% lactic acid in 10 mL of milk. Next, 0.1 mL (1%) of GODO-TCF and 0.9 mL, or 1 mL (10%) of sterile water was added to the 10 mL of milk, and the resultant preparation was left to stand at 43°C for 60 minutes. Meanwhile, as a comparative example, in order to inactivate the protease activity in GODO-TCF, GODO-TCF was diluted by 4 times with distilled water, and then the diluted preparation was subj ected to heat treatment at 100°C for 120 minutes. Into milk of which the pH had been adjusted to 5.9, 0.4 mL (1% in terms of before dilution) of GODO-TCF to which heat treatment had been performed after dilution, and 0.6 mL of sterile water were added.

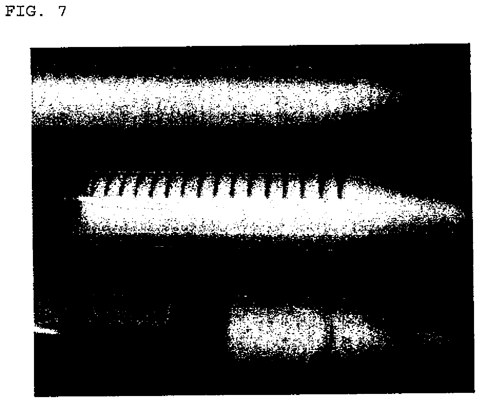

The results are shown in Fig. 7. As shown in Fig. 7, in a case where GODO-TCF in which protease had been inactivated was added, milk was not coagulated, whereas the milk to which 1% and 10% of GODO-TCF had been added was coagulated.

### Example 2

10 g of milk (Oishii Gyunyu, manufactured by Meiji Co., Ltd.) was weighed out in a 15-mL assist tube, and 0.2 g (2%, w/w) of skim milk was added to the milk, and the resultant preparation was thoroughly stirred and dissolved, and was boiled for 5 minutes in boiling water to sterilize. Then, cooled milk was preincubated in 43°C, and 0.1 mL (1%, w/v) of TCF sterilized by filtration through a 0.2-µm filter, and 0.3 mL of sterilized MilliQ water were aseptically added in a clean bench. As a control, one obtained by adding 1.2 mL of the TCF diluted by 4 times with MilliQ water and boiled in boiling water for 2 hours, and one obtained by adding 1.2 mL of sterilized MilliQ water were prepared. Then, in a clean bench, 0.5 g (5%, w/w) of a starter (Bulgaria Yogurt, manufactured by Meiji Co., Ltd.) was aseptically added to each tube, and the resultant preparation was stirred gently by inverting the tube 2 to 3 times. Seven tubes were prepared for each sample, and the fermentation was stopped every one hour from immediately after the addition of a starter until 6 hours later, and the pH was measured.

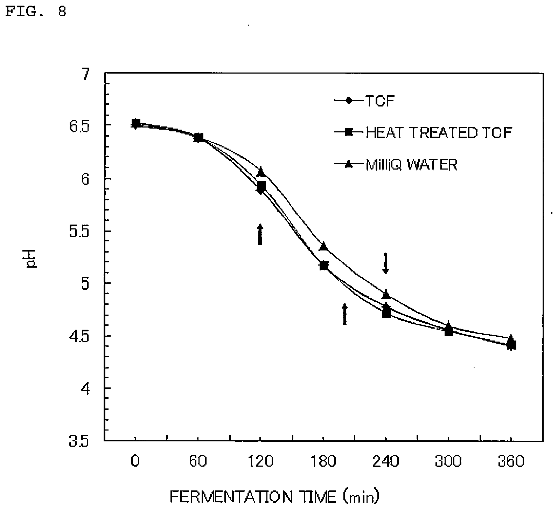

As a result, as shown in Fig. 8, it was found that in both controls of the heat-treated GODO-TCF (protease inactivated) and the MilliQ water addition, coagulation occurred in the vicinity of the isoelectric point (pl = 4.6) of the casein protein in milk, whereas in a case of the GODO-TCF addition, the coagulation occurred in the vicinity of pH 5.9.

### Example 3

### (Method)

55.8 g of milk (Oishii Gyunyu, manufactured by Meiji Co., Ltd.) was weighed out in a jam bottle (Jam 90ST, manufactured by Toyo Glass Co., Ltd.), and 1.2 g (2%, w/w) of skim milk was added to the milk, and the resultant preparation was thoroughly stirred and dissolved, and was boiled for 5 minutes in boiling water to sterilize. Then, cooled milk was preincubated at 43°C, and 6mL (1%, w/v) of the GODO-TCF sterilized by filtration through a filter having a pore size of 0.2 µm was aseptically added in a clean bench. As the blank, 0.6 mL (1%) of a 50 mM citrate buffer solution (pH 5.2, r.t.) was added. Then, 3 g (5%, w/w) of a starter (Bulgaria Yogurt, manufactured by Meiji Co., Ltd.) was aseptically added to each tube in a clean bench, the resultant preparation was stirred gently by inverting the tube 2 to 3 times, and the fermentation was conducted at 43°C for 4 hours. The fermented milk samples were subjected to texture analysis in each container.

For the analyzer, a creep meter RE-33005C (YAMADEN co., ltd.) was used. In the breaking strength analysis, the measurement was performed using a jig No. 3 under the conditions of 4°C, an amplifier magnification of 10, a storage pitch of 750, a measured strain rate of 60%, a measurement speed of 10 mm/sec, and automatic measurement of sample thickness. In the texture analysis under a non-breaking condition, the measurement was performed using a jig No. 3 under the conditions of 4°C, an amplifier magnification of 10, a storage pitch of 875, a measured strain rate of 5%, a measurement speed of 3 mm/sec, a return distance of 30 mm, and automatic measurement of sample thickness. In the texture analysis under a breaking condition, the measurement was performed using a jig No. 3 under the conditions of 4°C, an amplifier magnification of 10, a storage pitch of 875, a measured strain rate of 50%, a measurement speed of 10 mm/sec, a return distance of 30mm, and automatic measurement of sample thickness.

### <Data obtained from the texture profile (two-bite method)>

Hardness (H) : The maximum load on application. For semi-solid food, stress divided by jig cross-sectional area.

Brittleness (B) : The amount of juice drop at the time of the occurrence of partial destruction on compression.

Adhesive force (C) : The maximum load value at which adhesion at the time of the return tension after compression is observed. For semi-solid food, the jig cross-sectional area is taken into account.

Adherability (A3): The amount difficulty in separation in a case of having adhesiveness.

Cohesiveness (A2 / A1): The ratio and the deformation amount of compression energy between the first stroke and the second stroke.

Elasticity (T2 / T1): The ratio in the change amount reaching the maximum peak between the first stroke and the second stroke.

Gum property (hardness × cohesiveness): Higher value indicates higher hardness, and it can be assumed to be rubbery.

Chewiness (hardness × cohesiveness × elasticity) : Higher value indicates that more energy is required for chewing.

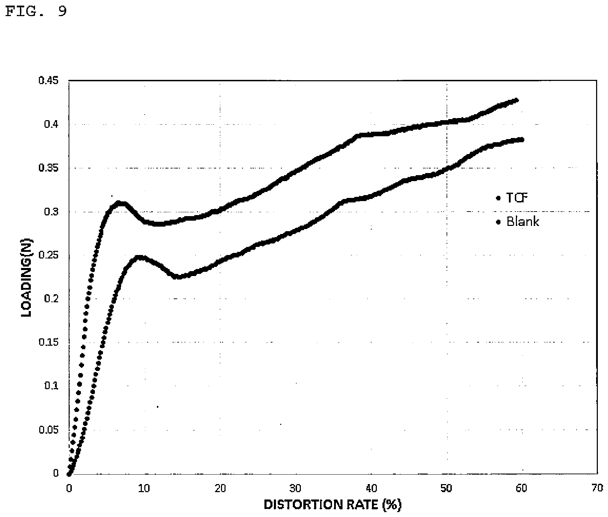

In the breaking strength analysis, in the GODO-TCF added sample, the breaking load was increased by 0.03 N and the breaking strain rate was decreased by 4.4% as compared with those of the additive-free sample at a breaking point (Fig. 9, and Table 1).

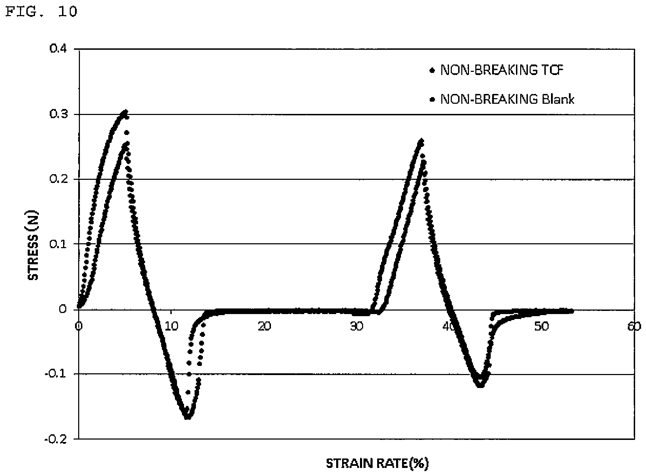

In the texture analysis of non-breaking (Fig. 10), the maximum stress during surface compression ([1] and [3]) was increased by 0.5 N in the GODO-TCF added sample. As to the cohesiveness, and the adherability ([2]), the values were decreased in the GODO-TCF added sample.

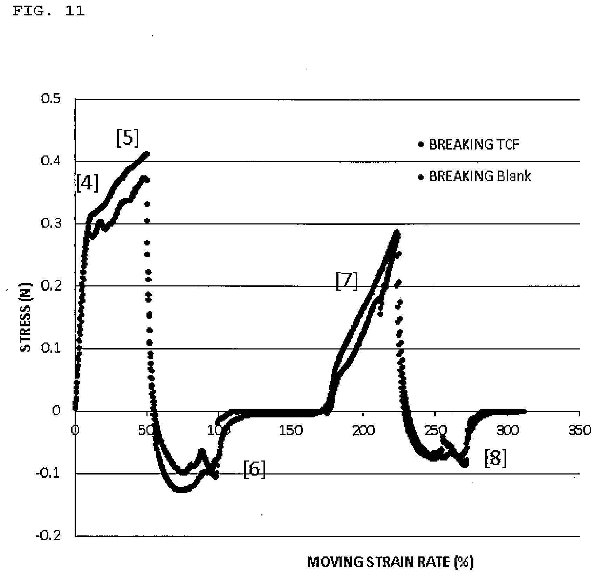

In the texture analysis of breaking (Fig. 11), in the GODO-TCF added sample, the stress at the time of breaking was increased ([4]). Waveforms ([5] and [7]) showing the breaking stress inside the curd are relatively smooth in the GODO-TCF added sample, whereas in the additive-free sample, a breaking point was able to be confirmed in several places. Moreover, in the cohesiveness and the adherability ([6] and [8]), the values were higher in the GODO-TCF added sample as compared with those in the additive-free sample. In the waveforms of the adherability ([6] and [8]) at the time of being pulled out, it was found that the GODO-TCF added sample was smoothly pulled out, whereas in the additive-free sample, the waveform was disturbed, and resistance was applied.

**[Table 1]**

| Measurement values of breaking strength analysis | | | |
|---|---|---|---|
| | Breaking load (N) | Breaking energy (J/m³) | Breaking strain rate (%) |
| TCF added | 0.316 | 375.47 | 6.64 |
| Blank | 0.282 | 291.32 | 11.2 |

### Example 4

### (Method)

In accordance with the fermented milk preparation method of Example 3, fermented milk samples were prepared on a 200 g scale. As the Sumizyme C (cellulase preparation, manufactured by Shin-Nihon Chemical Co., Ltd.), a preparation diluted so as to have chymosin activity equivalent to that of GODO-TCF by the chymosin activity method described in (3) of Production Example 1 was used. For the Blank, 0.6 mL (1%) of a 50 mM citrate buffer solution (pH 5.2, r.t.) was added.

As the analyzer, a taste sensing device TS-5000Z (Insent) was used. For the sample for taste sensor analysis, one obtained by diluting a fermented milk sample by 2 times with purified water (w/w) was used. As a control for analysis, one obtained by similarly diluting Bulgaria Yogurt (MeijiCo., Ltd.) was used. For the sensor used for analysis, a blend membrane, a plus membranes, a minus membrane, and GL1 (sweet taste sensor) were used.

The results of the taste sensor analysis are shown in Table 2. Each analysis value is expressed as a relative value based on the analysis values of Bulgaria Yogurt.

In the 1% GODO-TCF added sample, sweetness, and acidic bitterness were lower than those in the samples of control, 0.1% GODO-TCF, citrate buffer solution added. In the 0.1% GODO-TCF added sample, the results were almost the same as those of the citrate buffer solution added sample. In the Sumizyme C added sample, acidic bitterness, and a aftertaste from acidic bitterness were higher.

From the results described above, it was found that when the influence of salts and buffer components was removed, changes in tastes other than the acidic bitterness and sweetness were not generated. In addition, it was found that bitterness was hardly generated even when the GODO-TCF derived protease was allowed to act on a milk protein.

**[Table 2]**

| Analysis values of each taste by taste sensor | | | | | | | | | |
|---|---|---|---|---|---|---|---|---|---|
| Sample | Sourness | Acidic bitterness | Astringency | Mineral-based bitter taste 2 | Aftertaste from acidic bitterness | Aftertaste from astringency | Umami | Richness | Sweetness |
| Bulgaria (Control) | 0 | 0 | 0 | 0 | 0 | 0 | 0 | 0 | 0 |
| 1% TCF | -0.09 | -0.32 | 0.09 | 0.05 | 0 | 0.14 | -0.03 | 0.56 | -0.41 |
| 0.1 %TCF | 0.05 | -0.03 | -0.09 | 0.06 | 0.01 | 0.04 | -0.02 | 0.35 | 0.11 |
| Citrate buffer solution (Blank) | -0.14 | 0.12 | 0 | 0.09 | 0.07 | 0.05 | 0.03 | 0.48 | 0.19 |
| Sumizyme | -0.41 | 0.19 | 0.13 | 0.12 | 0.27 | 0.14 | 0.17 | 0.74 | -0.04 |

## Claims

1. A dairy product, comprising: a milk-derived raw material containing casein; and a protein having caseinolytic activity consisting of an amino acid sequence having 90% or more identity to the amino acid sequence represented by SEQ ID NO: 1.

2. The dairy product according to claim 1, wherein the protein is a protein derived from a microorganism belonging to the genus Trichoderma.

3. The dairy product according to claim 1 or 2, further comprising lactic acid bacteria or yeast.

4. The dairy product according to any one of claims 1 to 3, wherein the dairy product is fermented milk or cheese.

5. The dairy product according to any one of claims 1 to 4, wherein the dairy product has a breaking stress in a two-bite method of 5,500 Pa or more, and a breaking adhesion of 800 J/m³ or more, wherein both parameters are determined according to a method as set forth in Example 3.

6. The dairy product according to any one of claims 1 to 5, not comprising a thickening agent, a stabilizer, or a gelling agent.

7. A method for producing a dairy product, comprising: adding a protein having caseinolytic activity consisting of an amino acid sequence having 90% or more identity to the amino acid sequence represented by SEQ ID NO: 1, and lactic acid bacteria to a milk-derived raw material containing casein; and fermenting a mixture of the protein and the lactic acid bacteria with the milk-derived raw material.

## Patentansprüche

1. Ein Milchprodukt, umfassend: einen aus Milch gewonnenen Rohstoff, der Kasein enthält; und ein Protein mit kaseinolytischer Aktivität bestehend aus einer Aminosäuresequenz mit einer Identität von 90% oder mehr zu der Aminosäuresequenz, dargestellt durch SEQ ID NO: 1.

2. Das Milchprodukt nach Anspruch 1, wobei das Protein ein Protein ist, das von einem Mikroorganismus stammt, der zur Gattung Trichoderma gehört.

3. Das Milchprodukt nach Anspruch 1 oder 2, ferner umfassend Milchsäurebakterien oder Hefe.

4. Das Milchprodukt nach einem der Ansprüche 1 bis 3, wobei das Milchprodukt fermentierte Milch oder Käse ist.

5. Das Milchprodukt nach einem der Ansprüche 1 bis 4, wobei das Milchprodukt eine Bruchspannung in einem Zwei-Biss-Verfahren von 5.500 Pa oder mehr und eine Bruchhaftung von 800 J/m³ oder mehr aufweist, wobei beide Parameter nach einem Verfahren wie in Beispiel 3 dargelegt bestimmt werden.

6. Das Milchprodukt nach einem der Ansprüche 1 bis 5, das kein Verdickungsmittel, keinen Stabilisator und kein Geliermittel enthält.

7. Verfahren zur Herstellung eines Milchprodukts, umfassend: Zugabe eines Proteins mit kaseinolytischer Aktivität, das aus einer Aminosäuresequenz mit 90% oder mehr Identität zu der durch SEQ ID NO: 1 dargestellten Aminosäuresequenz besteht, und Milchsäurebakterien zu einem aus Milch gewonnen Rohstoff, der Kasein enthält; und Fermentieren einer Mischung des Proteins und der Milchsäurebakterien mit dem aus Milch gewonnenen Rohstoff.

## Revendications

1. Produit laitier comprenant: une matière première dérivée du lait contenant de la caséine; et une protéine ayant une activité caséinolytique consistant en une séquence d'acides aminés ayant une identité de 90 % ou plus avec la séquence d'acides aminés représentée par la SEQ ID NO : 1.

2. Produit laitier selon la revendication 1, dans lequel la protéine est une protéine dérivée d'un microorganisme appartenant au genre Trichoderma.

3. Produit laitier selon la revendication 1 ou 2, comprenant en outre une levure ou une bactérie d'acide lactique.

4. Produit laitier selon l'une quelconque des revendications 1 à 3, dans lequel le produit laitier est un lait fermenté ou un fromage.

5. Produit laitier selon l'une quelconque des revendications 1 à 4, dans lequel le produit laitier a une contrainte de rupture dans un procédé de double morsure de 5 500 Pa ou plus, et une adhérence à la rupture de 800 J/m³ ou plus, dans lequel les deux paramètres sont déterminés conformément à un procédé tel qu'indiqué dans l'Exemple 3.

6. Produit laitier selon l'une quelconque des revendications 1 à 5, ne comprenant pas d'agent épaississant, de stabilisant, ou d'agent gélifiant.

7. Procédé pour produire un produit laitier, comprenant : l'addition d'une protéine ayant une activité caséinolytique consistant en une séquence d'acides aminés ayant une identité de 90 % ou plus avec la séquence d'acides aminés représentée par la SEQ ID NO : 1, et d'une bactérie d'acide lactique, à une matière première dérivée du lait contenant de la caséine ; et la fermentation du mélange de la protéine et du ferment lactique avec la matière première dérivée du lait.
